# EUROPEAN PATENT APPLICATION

(11) **EP 3 556 861 A1**
(43) Date of publication of application: **23.10.2019**
(21) Application number: 17883434.7
(22) Date of filing: 18.12.2017
(51) Int. Cl.: C12Q 1/04, G01N 33/53, C07K 14/52, C12N 5/0781, C12N 5/0783, C12N 5/0786

(54) **METHOD FOR IN VITRO EARLY DIAGNOSIS OF POST-ORGAN-TRANSPLANTATION ANTIBODY MEDIATED REJECTION USING IgM-TYPE MEMORY B CELL DIFFERENTIATION CULTURE SYSTEM**

(30) Priority: 19.12.2016 JP 2016245749
(71) Applicant: Osaka University, Suita-shi, Osaka 565-0871 (JP)
(72) Inventor: TAKAHARA, Shiro, Suita-shi Osaka 565-0871 (JP); MATSUDA, Yoshiko, Suita-shi Osaka 565-0871 (JP)
(74) Representative: Vossius & Partner Patentanwälte Rechtsanwälte mbB
(86) International application number: PCT/JP2017/045267
(87) International publication number: WO 2018/117011

(57) **Abstract**

A means for detecting in vitro the presence or absence of antibody mediated rejection (ABMR) in patients after organ transplantation in a simple manner at an early stage not through biopsy of organs is provided. A method for detecting in vitro the presence or absence of antibody mediated rejection (hereinafter referred to as "ABMR") in patients after organ transplantation, comprising culturing peripheral blood sample from the patients in the presence of humoral factors from activated T cells and/or inflammatory cytokines, and detecting IgM-type DSA in the culture supernatant, wherein the detection of IgM-type DSA can be an index of early diagnosis of ABMR, and a kit used for said method.

## Description

### TECHNICAL FIELD

The present invention relates to a method for early diagnosis of antibody mediated rejection (hereinafter also referred to as "ABMR") after organ transplantation using IgM-type memory B cells. More specifically, the present invention relates to a method for detecting in vitro the presence or absence of antibody mediated rejection (ABMR) in patients after organ transplantation and a kit for detecting in vitro the presence or absence of ABMR in patients after organ transplantation.

### BACKGROUND ART

Organ transplantation is a very effective therapy for patients suffering from chronic organ failure in view of improvement of QOL and further in view of maintenance of life. However, in Japan, in comparison with the USA and Europe, organs provided are severely deficient and in case of kidney, many of patients suffering from chronic renal failure depend on dialysis treatment for maintenance of life. Furthermore, in case of liver, heart and lung transplantation, destruction of these organs directly leads to death and there are only limited opportunities for receiving organ donation. Even if patients are lucky enough to receive organ donation, they can hardly hope to receive another organ donation. Thus, necessity for retaining the function of transplanted organ for a long period of time is higher in Japan than in the USA and Europe where medical transplantation is more diffused. On the other hand, although short-term prognosis of transplantation has been markedly improved, long-term performance from 5 to 10 years onward relating to antibody mediated rejection (ABMR) has not been improved. For ABMR, since microvessels of transplanted organs are irrevocably damaged with the progress of the disease, early diagnosis is necessary. At present, only tissue biopsy is available for early diagnosis but is highly invasive and thus is difficult for frequent performance. Therefore, there is a need for the development of a new method of immune monitoring which is readily applicable to clinics, is simple, economic and highly reproducible.

In recent years, by development of a highly sensitive method for measuring an HLA antibody (analysis by Luminex technology using Single antigen beads; LABScreen etc.), relationship between Donor Specific HLA Antibody (hereinafter also referred to as "DSA") and ABMR has been elucidated (Non-patent reference 1, Non-patent reference 2). Thus, for seeking the possibility of ABMR regulation, post-transplantation serum monitoring has been performed but its effectiveness is not known. One of the reasons is the limit of DSA detection. At an early stage, DSA is adsorbed to a graft and thus is not likely to be detected in peripheral blood (Non-patent reference 3). Therefore, TTS guideline does not recommend the measurement in sera one year after transplantation onward. It is only under the circumstances where DSA production is exceeding and DSA is overflowed from a graft or under the circumstances where blood flow to a graft is decreased that DSA is detected in peripheral blood. Therefore, there is a possibility that the progress of ABMR cannot precisely be monitored by DSA in sera. However, if DSA produced from antibody-producing cells (plasma cells) of patients could be detected in vitro, the produced DSA is not affected by its adsorption to transplanted organs and thus DSA can be detected at an earlier stage than in sera to allow for keener grasp of the progress of humoral immune reaction. Therefore, IgG DSA has been focused in view of antigen specificity and in vitro detection from supernatant of peripheral blood B cell culture has been attempted. In several laboratories, IgG DSA has been successfully detected from supernatant of peripheral blood B cell culture. However, since 30 ml or more of peripheral blood is necessary for detection and the obtained information is limited as compared to sera, clinical application has not yet been attained (Non-patent reference 4). Plasma cells are present in the bone marrow and the secondary lymphoid tissues but seldom in peripheral blood. Therefore, it is almost impossible to monitor DSA in peripheral blood.

Hitherto, as a method for diagnosing or monitoring the rejection of organ transplantation such as kidney transplantation, those methods based on genes differently expressed in subjects (Patent references 1, 2, 3, 4, 5, 6, 7, 8, 9, 10, 11 and 12) and those methods based on polypeptide marker (Patent references 13, 14, 15, 16, 17 and 18) are known.

### [Prior Art]

### [Patent reference]

Patent reference 1: JP 2005-536230
Patent reference 2: JP 2007-520209
Patent reference 3: JP 2008-545444
Patent reference 4: JP 2008-545445
Patent reference 5: JP 2008-545446
Patent reference 6: JP 2009-195234
Patent reference 7: JP 2009-529340
Patent reference 8: JP 2009-532047
Patent reference 9: JP 2010-524427
Patent reference 10: JP 2011-515068
Patent reference 11: JP 2013-509883
Patent reference 12: JP 2016-531580
Patent reference 13: JP 2008-545130
Patent reference 14: JP 2011-522267
Patent reference 15: JP 2012-506537
Patent reference 16: JP 2012-506538
Patent reference 17: JP 2012-508382
Patent reference 18: JP 2012-510058

### [Non-patent reference]

Non-patent reference 1: Significance of qualitative and quantitative evaluations of anti-HLA antibodies in kidney transplantation. Transplant International 2011; 24(2); 150-157.
Non-patent reference 2: HLA antibodies and chronic rejection: From association to causation. Transplantation 2008; 86; 377-383.
Non-patent reference 3: Detection of donor-specific HLA antibodies before and after removal of a rejected kidney transplant. Transplant Immunology 2010; 22(3-4); 105-109.
Non-patent reference 4: Peripheral blood B cells producing donor-specific HLA antibodies in vitro. Hum Immunol. 2009; 70(1) ;29-34.
Non-patent reference 5: Annual Progress Report from the Japanese Renal Transplant Registry: Number of Renal Transplantations in 2010 and a Follow-up Survey -3 2009; Japanese Society for Clinical Renal Transplantation. Japanese journal of transplantation 2010; 45; 608.
Non-patent reference 6: Long-term renal allograft survival in the United States: a critical reappraisal. Am J Transplant 2011; 11; 450-62.
Non-patent reference 7: APRIL promotes cell-cycle progression in primary multiple myeloma cells: influence of D-type cyclin group and translocation status. Blood 2011; 117(3); 890-901.
Non-patent reference 8: BAFF production by antigen-presenting cells provides T cell co-stimulation. Int. Immunol 2004; 16(3); 467-475.
Non-patent reference 9: An in vitro model of differentiation of memory B cells into plasmablasts and plasma cells including detailed phenotypic and molecular characterization. Blood 2009; 114(25); 5173-81.
Non-patent reference 10: Clinical Relevance of HLA Antibody Monitoring after Kidney Transplantation. Journal of Immunology Research volume 2014; Article ID 845040.
Non-patent reference 11: Human leukocyte antigen antibodies and chronic rejection: from association to causation. Transplantation 2008; 86(3):377-83.
Non-patent reference 12: All chronic rejection failures of kidney transplants were preceded by the development of HLA antibodies. Transplantation 2002; 74(8):1192-4.
Non-patent reference 13: Regulation of CXCR3 and CXCR4 expression during terminal differentiation of memory B cells into plasma cells. Blood 2005; 105(10): 3965-71.
Non-patent reference 14: Unique properties of memory B cells of different isotypes. Immunol Rev 2010; 237(1): 104-16.
Non-patent reference 15: Early differentiated CD138 (high) MHCII+ IgG+ plasma cells express CXCR3 and localize into inflamed kidneys of lupus mice. PLoS One 2013; 8(3).
Non-patent reference 16: Memory B cells in the lung participate in protective humoral immune responses to pulmonary influenza virus reinfection. Proc Natl Acad Sci U S A2012; 109(7): 2485-90.
Non-patent reference 17: Human blood IgM "memory" B cells are circulating splenic marginal zone B cells harboring a prediversified immunoglobulin repertoire. Blood 2004; 104(12): 3647-54.
Non-patent reference 18: Repression of the transcription factor Bach2 contributes to predisposition of IgG1 memory B cells toward plasma cell differentiation. Immunity 2013; 25;39(1): 136-47.
Non-patent reference 19: Antigen-specific memory B cell development. Annu Rev Immunol 2005; 23: 487-513.
Non-patent reference 20: Impact of IgM and IgG3 anti-HLA alloantibodies in primary renal allograft recipients. Transplantation 2014; 97(5): 494-501.
Non-patent reference 21: Why do we need IgM memory B cells?. Immunol Lett 2013; 152(2): 114-20.
Non-patent reference 22: In-vitro derived germinal centre B cells differentially generate memory B or plasma cells in vivo. Nat Commun 2011; 2; 465.

### DISCLOSURE OF THE INVENTION

### (Technical Problem to be Solved by the Invention)

As stated above, relationship between DSA and ABMR has been elucidated and serum DSA monitoring after transplantation has been performed. However, at an early stage, DSA is adsorbed to a graft and thus is not likely to be detected in peripheral blood. Serum DSA after transplantation could only be detected under the circumstances where DSA production is exceeding and DSA is overflowed from a graft, namely after ABMR has considerably progressed. Also, detection of IgG-type DSA in in vitro peripheral B cell culture supernatant has been attempted. However, it is known that IgG-type memory B cells are localized in the secondary lymphoid tissues or inflammatory tissues after antigen sensitization in preparation for the secondary antigen invasion. Therefore, it is anticipated that IgG-type memory B cells are highly localized in the secondary lymphoid tissues (for instance, internal and external iliac artery lymphatic tissue in case of renal transplantation) around the portion where organs are transplanted and are unlikely to circulate in peripheral blood. Furthermore, since 30 ml or more of peripheral blood is necessary for detection of IgG-type DSA and the obtained information is limited as compared to sera, clinical application has not yet been attained.

Thus, up till the present, a method for early monitoring of serum DSA after transplantation has not yet been established. Also, there were a problem of heavy burden of patients since 30 ml or more of peripheral blood is necessary for detection of IgG-type DSA and a problem that the obtained information is limited as compared to sera and hence clinical application has not yet been attained.

### (Means for Solving the Problems)

The present inventors, using peripheral blood mononuclear cells of patients, have established an in vitro system for differentiating and culturing IgM-type memory B cells to antibody-producing cells and have found that the production of IgM-type DSA in sera after transplantation could be monitored at an early stage by analyzing antibodies in the supernatant.

It is known that IgG-type memory B cells are localized in the secondary lymphoid tissues or inflammatory tissues after antigen sensitization in preparation for the secondary antigen invasion. Therefore, it is anticipated that IgG-type memory B cells are highly localized in, for instance, internal and external iliac artery lymphatic tissue in case of renal transplantation and are unlikely to circulate in peripheral blood. It is also reported that expression of chemokine receptors etc. is involved in tissue localization. On the other hand, for IgM-type memory B cells corresponding to DSA, expression of such chemokine receptors has not been reported. As such, the present inventors anticipated that IgM-type memory B cells may circulate in peripheral blood at higher frequency. However, IgM-type antibodies, after sensitization, can be detected in sera for a short period of time but thereafter cannot be detected. Therefore, for detecting IgM-type antibodies in culture supernatant, IgM-type memory B cells, which are ordinarily present as membrane cells, needed to be differentiated into secretory cells (antibody-producing cells) in vitro. In the actual clinic, it is reported that IgM-type DSA is detected in sera only in cases of rejection with advanced disease conditions. The present inventors therefore anticipated that inflammatory cytokines or humoral factors from activated T cells may play an important role in differentiation of IgM-type memory B cells into secretory cells. Thus, the present inventors added Protein kinase C, T cell receptor stimulator to the culture condition of peripheral blood mononuclear cells to stimulate T cells or macrophages so as to accelerate the inflammatory cytokines and the production of humoral factors from activated T cells. As a result, stable detection of IgM-type DSA in culture supernatant became possible.

Thus, the present invention includes the followings.
[1] A method for detecting in vitro the presence or absence of antibody mediated rejection (ABMR) in patients after organ transplantation, comprising culturing peripheral blood sample from the patients in the presence of humoral factors from activated T cells and/or inflammatory cytokines, and detecting IgM-type anti-donor HLA antibody (Donor Specific HLA Antibody; hereinafter referred to as "DSA") in the culture supernatant, wherein the detection of IgM-type DSA can be an index of early diagnosis of ABMR.
[2] The method of [1] wherein culturing in the presence of humoral factors from activated T cells and/or inflammatory cytokines is carried out by addition of healthy adult mononuclear cell culture supernatant.
[3] The method of [1] or [2] wherein the organ transplantation is renal transplantation.
[4] The method of any of [1] to [3] wherein culturing peripheral blood sample from the patients is carried out under culture conditions suitable for B cells.
[5] A kit for detecting in vitro the presence or absence of antibody mediated rejection (ABMR) in patients after organ transplantation, comprising a means for culturing peripheral blood sample from the patients in the presence of humoral factors from activated T cells and/or inflammatory cytokines, and a means for detecting IgM-type DSA in the culture supernatant, wherein the detection of IgM-type DSA can be an index of the presence of ABMR.
[6] The kit of [5] wherein the means for culturing peripheral blood sample from the patients in the presence of humoral factors from activated T cells and/or inflammatory cytokines comprises healthy adult mononuclear cell culture supernatant.
[7] The kit of [5] or [6] wherein the organ transplantation is renal transplantation.
[8] The kit of any of [5] to [7] wherein the means for culturing peripheral blood sample from the patients is a means for culturing peripheral blood sample from the patients under culture conditions suitable for B cells.

### EFFECTS OF THE INVENTION

In accordance with the present invention, the presence or absence of ABMR in patients after organ transplantation can be detected in vitro at an early stage. Tissue biopsy from a transplanted organ, the only method for diagnosis of ABMR hitherto performed, was highly invasive and was difficult to perform frequently. In accordance with the present invention, diagnosis of ABMR can be carried out non-invasively with a small amount of peripheral blood.

Detection of IgM-type DSA in culture supernatant of peripheral blood mononuclear cells in accordance with the present invention, as compared to detection of IgG-type DSA in sera, has diagnostic advantages that the detection is possible with as small an amount of 8 ml of peripheral blood, and that IgM-type DSA is detected earlier than IgG-type DSA since IgG-type memory B cells are class-switched from IgM-type memory B cells. Also, from therapeutic point of view, IgM-type memory B cells are suggested to have a higher therapeutic response to the existing immunosuppressive therapy as compared to IgG-type memory B cells. As such, detection of IgM-type DSA antibodies in culture supernatant is expected to develop early diagnosis and therapy of ABMR to a new stage.

Furthermore, organ transplantation is very useful in view of improvement of QOL of patients or maintenance of life. Detection of IgM-type DSA in accordance with the present invention shows good cost performance in medical economy and can be expected to contribute saving of medical expense. Also, with detection of IgM-type antibodies in culture supernatant in accordance with the present invention, it is possible that the significance of detecting these antibodies is reaffirmed and its clinical application in the other fields (autoimmune diseases and the like) is expected.

### BRIEF DESCRIPTION OF DRAWINGS

Fig. 1 is a graph showing that an activation level of humoral immune reaction against HLA antigens affects an abundance ratio of IgG-type memory B cells or IgM-type memory B cells corresponding to HLA antigens present in peripheral blood.
Fig. 2A is a graph showing that IgM-type HLA antibodies detected in culture supernatant of peripheral blood mononuclear cells had HLA antibody specificity in conformity with that of IgG-type antibodies in sera and comprised antibodies against donor antigens.
Fig. 2B is a graph showing that IgM-type HLA antibodies detected in culture supernatant of peripheral blood mononuclear cells had HLA antibody specificity in conformity with that of IgG-type antibodies in sera and comprised antibodies against donor antigens. In Fig. 2B, Sup shows culture supernatant of peripheral blood mononuclear cells.
Fig. 3 is a graph showing that humoral factors produced from activated T cells play an important role in differentiation of IgM-type memory B cells into plasma cells.
Fig. 4 is a graph showing that IgM-type memory B cells are class-switched to IgG-type memory B cells by antigen stimulation from B cell receptor.
Fig. 5 is a graph showing the relationship between IgM-type memory B cells and IgG-type memory B cells.
Fig. 6 is a graph showing the results obtained by cross-linking IgM-type B cell receptor on the surface of B cells to stimulate IgM-type memory B cells in place of antigen stimulation.
Fig. 7 is a graph showing the comparison of IgG/IgM DSA in sera.
Fig. 8 is a graph showing that significantly more IgG and IgM can be detected by adding PHA-L and PMA to the culture condition of peripheral blood mononuclear cells (PBMC).
Fig. 9 is a graph showing that IgM DSA was detected in the culture supernatant immediately after transplantation, thereafter IgG DSA was detected in sera on Day 8 after transplantation, and the patient was diagnosed as ABMR on Day 9 after transplantation.
Fig. 10 is a graph showing that IgM DSA was detected in PBMC culture supernatant obtained on Day 8 after transplantation and IgG DSA was detected in sera on Day 28 after transplantation.
Fig. 11 is a graph showing that IgM DSA was detected in PBMC culture supernatant obtained on Day 67 after transplantation and IgG DSA was detected in sera on Day 133 after transplantation.
Fig. 12A is a graph showing that, when medication of standard immunosuppressive agents was initiated at the time of transplantation, a detection amount of IgG DSA was not changed before and after transplantation whereas a detection amount of IgM DSA was markedly decreased after transplantation.
Fig. 12B is a graph showing that, when medication of standard immunosuppressive agents was initiated at the time of transplantation, a detection amount of IgG DSA was not changed before and after transplantation whereas a detection amount of IgM DSA was markedly decreased after transplantation.

### BEST MODE FOR CARRYING OUT THE INVENTION

The present invention is characterized by that IgM-type memory B cells corresponding to DSA are differentiated and induced into secretory cells in vitro and IgM-type DSA is detected in culture supernatant, to be aimed for application to early diagnosis and treatment of antibody mediated rejection (ABMR).

Naive B cells are firstly differentiated into IgM-type memory B cells by antigen stimulation in the germinal center and are then class-switched to IgG-type memory B cells by further antigen stimulation. IgM-type memory B cells circulate in peripheral blood whereas IgG-type memory B cells move to, and are localized in, the secondary lymphoid tissues or inflammatory tissues in preparation for the secondary antigen invasion.

It is confirmed in vitro that IgM-type memory B cells corresponding to a donor antigen circulate in peripheral blood from at the point of low reactivity of antigen/antibody after sensitization and are class-switched when humoral immune reaction to the donor antigen is accelerated. Therefore, it is expected that IgM-type DSA in culture supernatant is detected at an earlier stage than IgG-type DSA in sera.

It was suggested that IgM-type memory B cells corresponding to DSA circulate in peripheral blood from at the point when humoral immune reaction to a donor antigen is not accelerated, i.e. at a comparatively early stage of ABMR. On the other hand, IgG-type memory B cells are usually localized in the secondary lymphoid tissues or inflammatory reaction and a frequency of its circulation in peripheral blood is lower than that of IgM-type memory B cells. It was thus anticipated that IgG-type memory B cells could firstly be detected in culture supernatant of peripheral blood B cells under the circumstances where ABMR was progressed or transplantation has been rejected.

Although IgG-type DSA has attracted attention in view of antigen/antibody specificity, since as much as 30-40 ml of peripheral blood is necessary for detection of IgG-type DSA in culture supernatant of peripheral blood B cells and the obtained information is limited as compared to sera, clinical application has not yet been attained.

The present inventors focused on IgM-type DSA viewing that IgM-type DSA can be detected in culture supernatant at an earlier stage than IgG-type DSA in sera since IgG-type memory B cells are surely differentiated from naive B cells via IgM-type memory B cells and that, in relation to the expression of chemokine receptor, IgM-type DSA is not localized in the secondary lymphoid tissues or inflammatory tissues and is highly likely to circulate in peripheral blood as compared to IgG-type DSA. In addition to antigen specificity, the reason why IgM-type DSA has not hitherto attracted attention is its low detection rate in sera and therefore IgM-type DSA could be detected only in limited cases where rejection has advanced. The reason is that IgM-type memory B cells are present as membrane cells and are thought to be unlikely to be differentiated into secretory cells. The results of the present inventors' study suggested that humoral factors from activated T cells and inflammatory cytokines may play an important role in differentiation of membrane cells into secretory cells. Thus, the present inventors have found that the in vitro culture of IgM-type memory B cells under conditions where these humoral factors are supplemented allows for effective differentiation and induction of IgM-type memory B cells corresponding to DSA into antibody-producing cells. Besides, the method of the present invention can detect IgM-type DSA in culture supernatant with as low an amount of 8 ml of peripheral blood and therefore can be performed in a less invasive manner as compared to the conventional diagnosis of ABMR such as renal biopsy. As such, the in vitro assay system of the present inventors, by taking advantage of characteristics of IgM-type memory B cells, can be a breakthrough means for diagnosing ABMR more easily and at an earlier stage.

In accordance with the present invention, the culture of peripheral blood from patients is carried out in the presence of humoral factors from activated T cells and/or inflammatory cytokines. With the culture, IgM-type memory B cells contained in samples are differentiated into secretory cells to thereby facilitate the detection of IgM-type DSA in culture supernatant. The culture in the presence of humoral factors from activated T cells and/or inflammatory cytokines can be performed by addition of healthy adult mononuclear cell culture supernatant.

Hitherto, CD40 ligand feeder cells have been used for the culture of B cells. However, since CD40 Ligand is contained in humoral factors produced from activated T cells, the assay system of the present invention does not include CD40 ligand feeder cells. Furthermore, these Feeder cells accelerate class-switch of IgM-type memory B cells and thus may possibly hamper evaluation of an abundance ratio of antibodies produced from IgM-type memory B cells and from IgG-type memory B cells under physiological conditions. In accordance with the present invention, an abundance ratio of IgM/IgG memory B cells is evaluated under more physiological conditions by the culture under more physiological conditions.

In accordance with the present invention, it was elucidated that humoral factors from activated T cells and inflammatory cells may play an important role in differentiation and proliferation of memory B cells. Therefore, the present inventors tried to culture intact peripheral blood mononuclear cells which were supplemented with cytokines that activate T cells and inflammatory cells. Hitherto, with the culture of intact peripheral blood mononuclear cells, survival maintenance and differentiation/proliferation of B cells were difficult since a growth rate and a survival rate are different from each other between T cells and B cells and thus T cells alone explosively differentiate and proliferate to thereby consume cytokines in the culture medium. Therefore, in accordance with the present invention, the culture was conducted under conditions favorable for B cells [Iscove's modified Dulbecco's medium (Sigma-Aldrich) with 10% fetal calf serum (Thermo Scientific HyClone, Logan, UT, USA) supplemented with 50 µg/ml human transferrin-selenium and 5 µg/ml human insulin (Gibco/Invitrogen Co., Carlsbad, CA, USA)].

Support by direct contact between cells plays an important role in differentiation and proliferation of memory B cells. The outcome can be obtained with a smaller amount of peripheral blood (8 ml) by the culture of intact peripheral blood mononuclear cells (20-30 ml or more of peripheral blood needs be taken when the culture is done after isolation of B cells).

In sera, IgG DSA is produced after sensitization with a donor antigen irrespective of disease conditions. On the other hand, an abundance ratio of memory B cells in peripheral blood is determined by on-going exposure to an antigen. Therefore, in peripheral blood, an abundance ratio of memory B cells corresponding to a donor antigen (transplant) is higher than that of memory B cells corresponding to a non-donor antigen (blood transfusion history, pregnancy history, infection history, etc.). Thus, hitherto, the presence and absence of antibodies against a donor antigen needs be judged by Single antigen beads, but in peripheral blood, the sensitization with a donor antigen can be judged only with FlowPRA screening. Thus, by evaluating antibodies derived from memory B cells in peripheral blood, more clinical information relating to ABMR can be obtained only with a simple screening.

As specific embodiments in which the present invention can be applied, the following, for example, can be considered in case of renal transplantation.
(1) After renal transplantation, 8-16 ml of peripheral blood is taken immediately after transplantation and subsequently regularly (at an interval of 1 to 3 months) from patients and in vitro culture is carried out. Culture supernatant is analyzed and, in cases where IgM-type DSA (in particular, DSAs, C1q binding) is detected, a dose of an immune suppresser (cellcept) which accelerates apoptosis of DSA-specific IgM memory B cells and a dose of an immune suppresser (CNI) which suppresses class-switch are increased.
(2) After renal transplantation, peripheral blood (8-16 ml) is taken from cases on Week 3 and in vitro culture is carried out. Culture supernatant is analyzed to check the detection of IgM-type DSA (in particular, DSAs, C1q binding). In cases where IgM-type DSA is detected, a dose of cellcept and CNI is maintained. In cases where IgM-type DSA is not detected, a decreased dose of cellcept and early termination of CNI are set as a goal. A month later, reexamination is carried out and, based on the results, ensuing therapeutic strategy is determined.
(3) In cases before renal transplantation, 8-16 ml of peripheral blood is taken before transplantation and in vitro culture is carried out. Culture supernatant is analyzed. In cases where IgM-type DSA (in particular, SAs, C1q binding) is detected, it is foreseen that DSA-specific IgM memory B cells are class-switched to DSA-specific IgG memory B cells at an early stage by sensitization with a donor antigen at the time of transplantation, causing the onset of ABMR. Thus, by initiating before transplantation a dose of an immune suppresser (cellcept) which accelerates apoptosis of DSA-specific IgM memory B cells and a dose of an immune suppresser (CNI) which suppresses class-switch, the onset of ABMR at an early stage after transplantation is prevented.

The present invention also provides a kit for practicing the above-mentioned embodiments, i.e. a kit for detecting the presence or absence of antibody mediated rejection (ABMR) in patients after organ transplantation in vitro, comprising a means for culturing peripheral blood sample from the patients in the presence of humoral factors from activated T cells and/or inflammatory cytokines, and a means for detecting IgM-type DSA in the culture supernatant, wherein the detection of IgM-type DSA can be an index of the presence of ABMR.

The present invention is explained in more detail in case of renal transplantation with the following examples but is not limited thereto.

### [Example 1]

### Isolation and culture of peripheral blood mononuclear cells

Peripheral blood (8 ml) was taken from cases of HLA antigen sensitization after renal transplantation, recipients of renal transplantation. Mononuclear cells were isolated from peripheral blood using Ficoll-Hypaque density gradient (Sigma-Aldrich, St Louis, MO, USA) and cultured. The culture medium was Iscove's modified Dulbecco's medium (Sigma-Aldrich) supplemented with 10% fetal calf serum (FCS; Thermo Scientific HyClone, NYSE, TMO), 50 µg/ml human transferrin-selenium and human insulin (Gibco Invitrogen Co., Carlsbad, CA, USA) and, as cytokines, 50 ng/ml IL-21 (Miltenyi Biotech), 2.5 µg/ml phosphorothioate CpG-ODN 2006 (Invivogen), 2.5 µg/ml phytohemagglutinin (PHA)(Sigma), 15 ng/ml phorbol 12-myristate-13 acetate (PMA). Peripheral blood mononuclear cells were further cultured on 24-well flat-bottom plates (5 × 10⁵ cells/well) for 7 days.

### [Example 2]

### Isolation and culture of peripheral blood B cells

Peripheral blood (8 ml) was taken from cases of HLA antigen sensitization after renal transplantation, from which mononuclear cells were isolated and further, using MACS separation (Miltenyi Biotec GmbH, Bergisch Gladbach, Germany), CD19-positive B cells were isolated. A basal medium was the same as that of mononuclear cells and was supplemented with 50 ng/ml IL-21, 2.5 µg/ml phosphorothioate CpG-ODN 2006, and 50 ng/ml histidine-tagged soluble recombinant human CD40L (R&D Systems, Minneapolis, MN, USA) as cytokines. Furthermore, as healthy adult mononuclear cell culture supernatant, peripheral blood mononuclear cells, where CD19-positive B cells were removed, taken from 10 healthy men without blood transfusion history were cultured on 24-well flat-bottom plates (1 × 10⁶ cells/well) for 36 hours and culture supernatant was collected. The culture medium was RPMI1640 supplemented with 10% fetal calf serum (FCS; Thermo Scientific HyClone, NYSE, TMO) and 5 µg/ml phytohemagglutinin (PHA)(Sigma), 10 ng/ml phorbol 12-myristate-13 acetate (PMA). The healthy adult mononuclear cell culture supernatant was added at 5% to the culture condition of peripheral blood B cells on 24-well flat-bottom plates (1 × 10⁵ cells/well) for culture for 7 days.

### [Example 3]

### Analysis of culture supernatant of peripheral blood mononuclear cells

Using FlowPRAR Class I & II Screening Test (One Lamda), the production of IgG-type or IgM-type antibodies, Class I or Class II HLA antibodies in the culture supernatant was screened. Further, in positive cases, profile of HLA antibodies and the presence and absence of anti-donor HLA antibodies were analyzed in detail using Luminex technology.

### [Example 4]

### Analysis of clinical sample

Using patient samples from cases of HLA antigen sensitization, antibodies produced by the established in vitro culture system and antibodies in sera were compared to each other. Also, the results of analysis of donor HLA antibodies in the culture supernatant and actual clinical course, as well as the results of pathological tissue of renal biopsy, were compared to each other.

### [Example 5]

### Activation level of humoral immune reaction against HLA antigens affects abundance ratio of IgG-type memory B cells or IgM-type memory B cells corresponding to HLA antigens present in peripheral blood

Cases were divided into two groups, i.e. a group of cases with De novo DSA-positive ABMR(-) and with stable function of renal transplant (Group I), and a group of cases where ABMR is diagnosed by renal biopsy or ABMR led to destruction of renal transplant (Group II), and further a group of cases where no HLA antibodies were produced in sera after transplantation as a control group (Group III). The culture supernatant of peripheral blood mononuclear cells collected from these cases was analyzed for the respective IgG-type and IgM-type by Flow PRA.

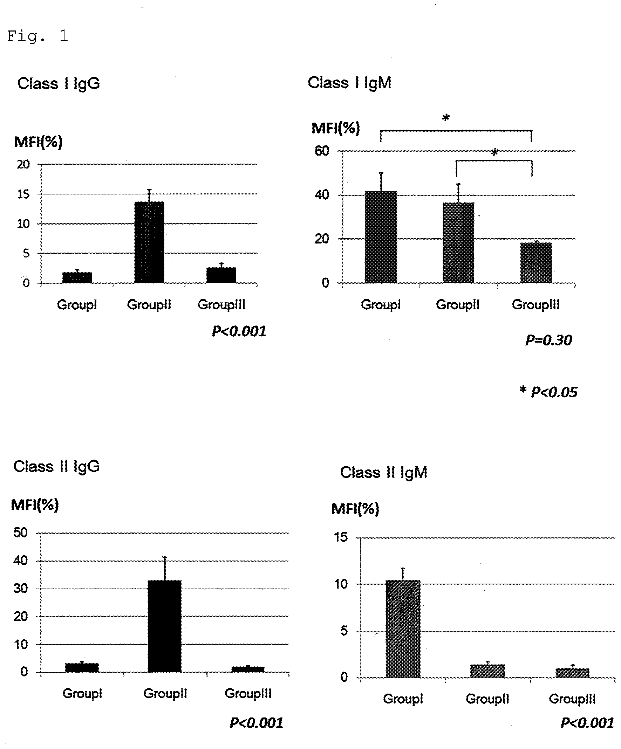

As a result, as shown in Fig. 1, IgM-type HLA antibodies were detected significantly more in Group I (class I; 31.76%, class II; 11.15%) than in Group III (class I; 18.1%, class II; 1.0%) for both Classes I and II (class I; p=0.014, class II; p=0.018). On the other hand, IgG-type HLA antibodies were detected significantly more in Group II than in Group III (class I; 2.04%, class II; 2.21%) for both Classes I and II (class I; p=0.01, class II; p=0.03).

### [Example 6]

### IgM-type HLA antibodies detected in culture supernatant of peripheral blood mononuclear cells had HLA antibody specificity in conformity with that of IgG-type antibodies in sera and comprised antibodies against donor antigens

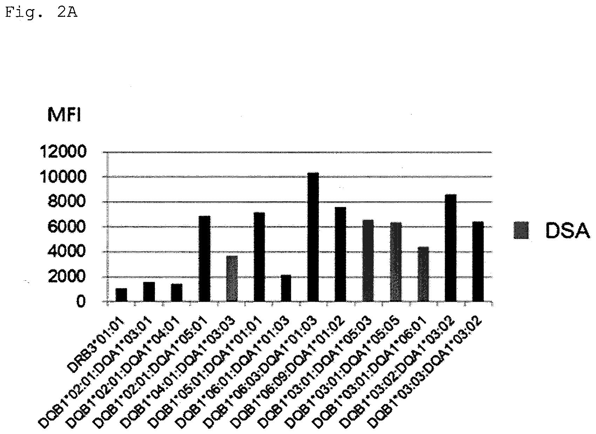

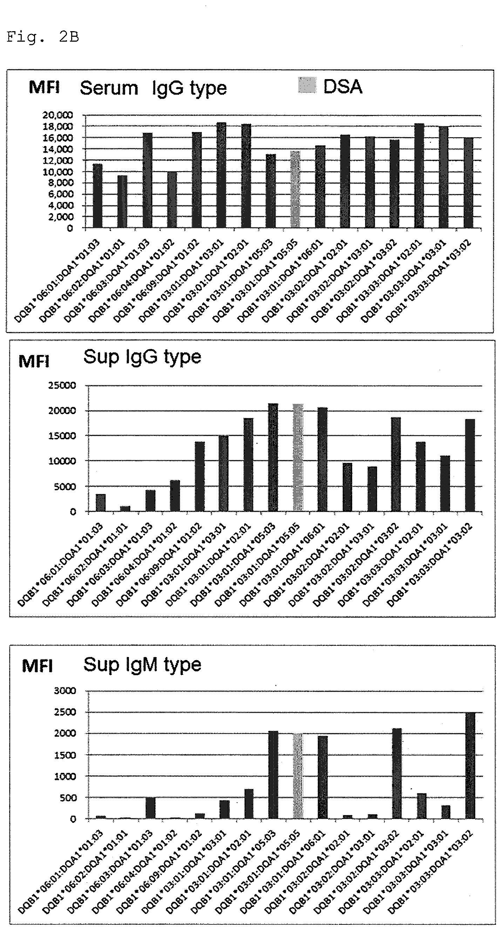

LABScreen was carried out in cases where IgM-type HLA antibodies were detected by Flow PRA screening in the culture supernatant of peripheral blood mononuclear cells. As a result, IgM-type DSA was also detected in cases with de novo DSA-positive and with stable renal function (Fig. 2A). On the other hand, DSA was detected as IgG-type and IgM-type in the group of rejection of renal transplantation. MFI values of IgG-type DSA in the culture supernatant were higher than those in sera, from which adsorption of IgG-type DSA to renal transplant was suspected (Fig. 2B).

### [Example 7]

### Humoral factors produced from activated T cells play important role in differentiation of IgM-type memory B cells into plasma cells

In cases where IgM-type HLA antibodies were detected in the culture supernatant of peripheral blood mononuclear cells, CD19-positive B cells were further isolated from peripheral blood. The cases were divided into a group of addition of the healthy adult mononuclear cell culture supernatant to the culture condition of B cells and a group of no addition. The culture supernatant was analyzed by Flow PRA screening and the results were compared.

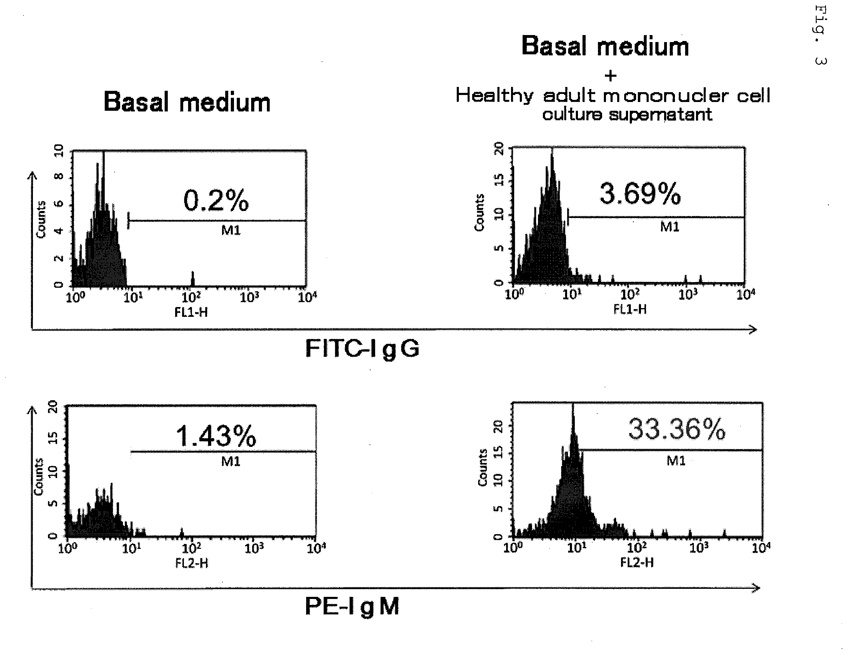

As a result, IgM-type HLA antibodies were detected only in the group of addition of the healthy adult mononuclear cell culture supernatant (Fig. 3). It is anticipated that humoral factors produced from activated T cells and inflammatory cytokines such as macrophages are contained in the supernatant. Therefore, it was suggested that these humoral factors may play an important role in differentiation of IgM-type memory B cells into antibody-producing cells.

### [Example 8]

### IgM-type memory B cells are class-switched to IgG-type memory B cells by antigen stimulation

In 10 cases where IgM-type HLA antibodies alone were detected in the culture supernatant, 5 µg/ml of Affini Pure F(ab)₂ Fragment Goat anti-Human IgM (Jackson Immuno Research) was added in place of antigen stimulation to the culture condition and the culture was carried out for 7 days. The culture supernatant was collected and analyzed by FLOW PRA screening.

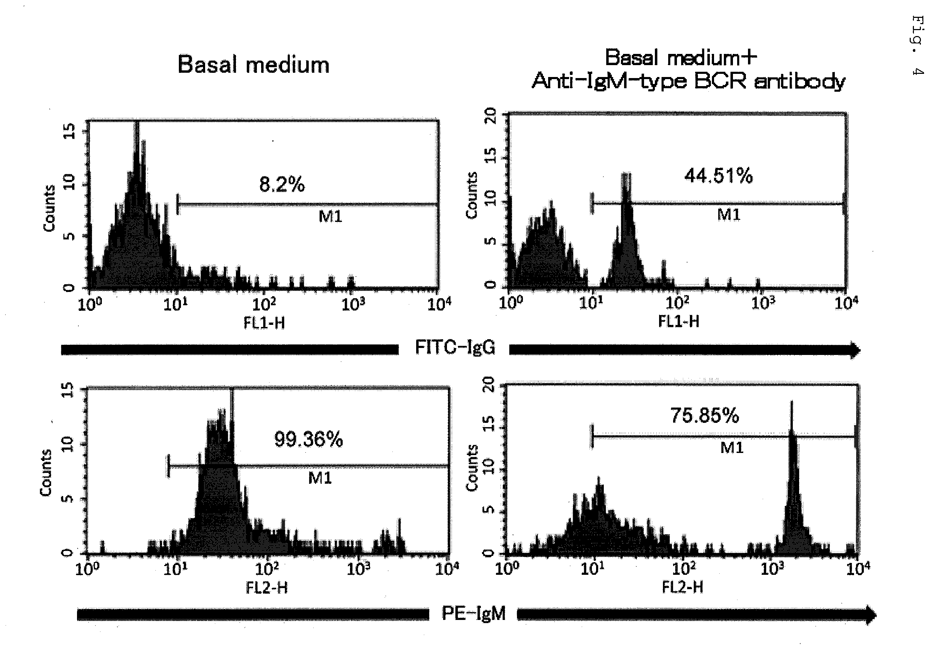

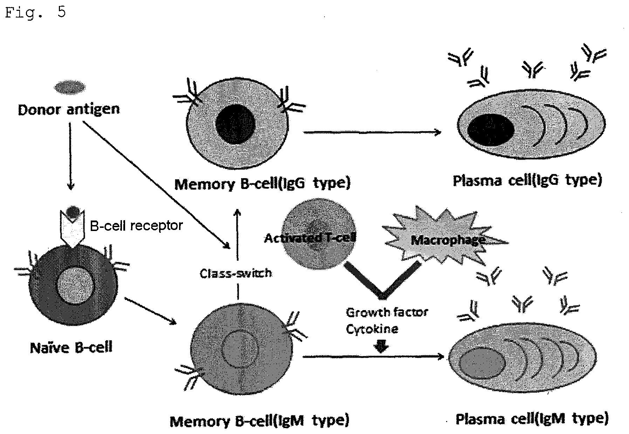

As a result, IgG-type HLA antibodies were detected in 7 cases. Furthermore, a portion of IgM-type HLA antibodies was detected as IgG-type in LABScreen (Fig. 4). Thus, it was suggested that class-switch of IgM-type memory B cells to IgG-type memory B cells was induced by antigen stimulation. Relationship between IgM-type memory B cells and IgG-type memory B cells is summarized in Fig. 5.

Detection of IgG/IgM DSA in the culture supernatant reflects acceleration of humoral immune reaction against a donor antigen. For further investigating relationship between antigen-specific IgM-type memory B cells and IgG-type memory B cells in the living body, analysis of EB virus-specific antibodies was conducted.

Peripheral blood mononuclear cells (5 × 10⁵ cells/well) taken from healthy adults were cultured on 24-well flat-bottom plates. For cytokines, IL-21 (50 ng/ml), CpG-ODN (2.5 µg/ml), phorbol myristate acetate (PMA) (2.5 µg/ml), phytohemagglutinin/leucoagglutinin (PHA-L)(15 µg/ml) were added as basal culture conditions. Furthermore, so as to mimic acceleration of antigen-antibody reaction via IgM BCR in vitro, the culture was also conducted under the conditions where Affini Pure F(ab)₂ Fragment Goat anti-Human IgM (5.2 µg/ml) was added for 7 days. Thereafter, the culture supernatant was collected and a concentration of IgG/IgM EBV antibodies was measured by ELISA. A concentration of IgG/IgM EBV antibodies in the culture supernatant was compared both under basal culture conditions and under the conditions where Affini Pure F(ab)₂ Fragment Goat anti-Human IgM was added.

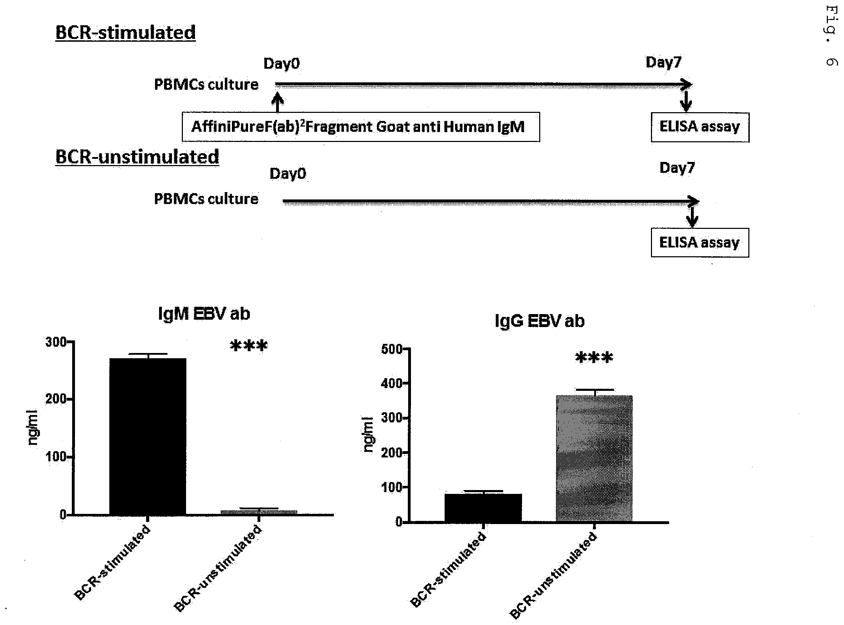

By cross-linking IgM-type B cell receptor on the surface of B cells in place of antigen stimulation, IgM-type memory B cells were stimulated. As a result, in the group of stimulation, IgG EBV antibodies were detected at a higher level whereas IgM decreased as compared to the group of no stimulation (Fig. 6). This proved that antigen-specific IgM-type memory B cells in peripheral blood class-switched to IgG-type memory B cells by antigen stimulation via B cell receptor.

### [Comparative Example 1]

### Comparison of IgG/IgM DSA in sera

Peripheral blood taken from healthy adults was centrifuged to separate sera. A concentration of IgG/IgM EBV antibodies in sera was measured by ELISA and compared.

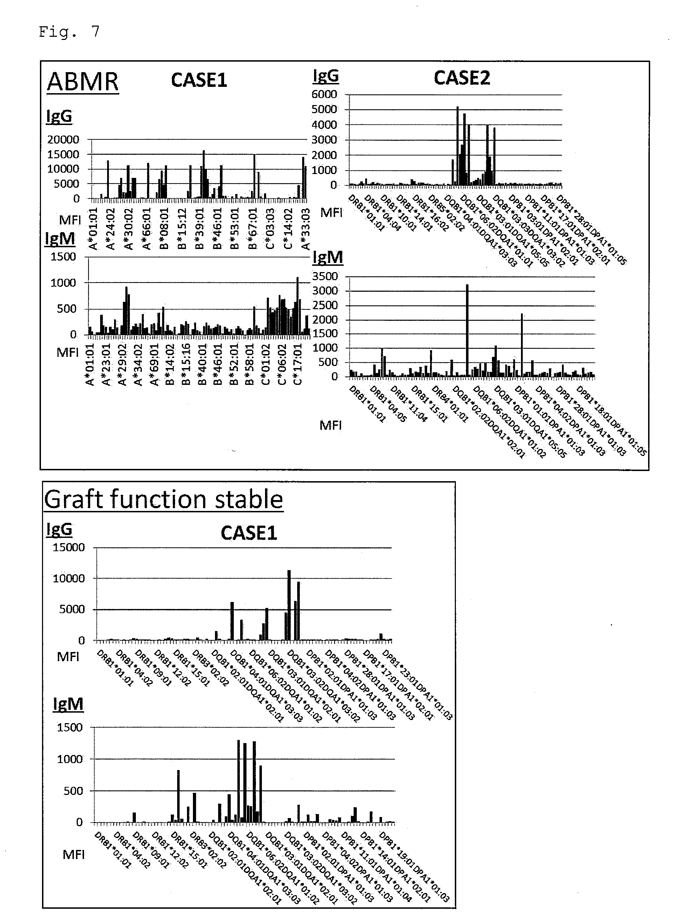

As a result, IgG DSA was detected from sera irrespective of disease conditions. On the other hand, a detection rate of IgM DSA was low and many pseudo-positive reactions were observed (Fig. 7).

### [Example 9]

### Significantly more IgG and IgM can be detected by adding PHA-L and PMA to culture condition of peripheral blood mononuclear cells (PBMC)

Peripheral blood mononuclear cells (5 × 10⁵ cells/well) taken from healthy adults were cultured on 24-well flat-bottom plates. For cytokines, IL-21 (50 ng/ml), CpG-ODN (2.5 µg/ml), phorbol myristate acetate (PMA)(2.5 pg/ml), phytohemagglutinin/leucoagglutinin (PHA-L)(15 µg/ml) were added as basal culture conditions and the culture was conducted for 7 days. The culture was also conducted for 7 days under conditions with no addition of PMA and PHA-L. Thereafter, the culture supernatant was collected and a concentration of IgG/IgM EBV antibodies was measured by ELISA. A concentration of IgG/IgM EBV antibodies in the culture supernatant was compared both under basal culture conditions and under the conditions with no addition of PMA and PHA-L.

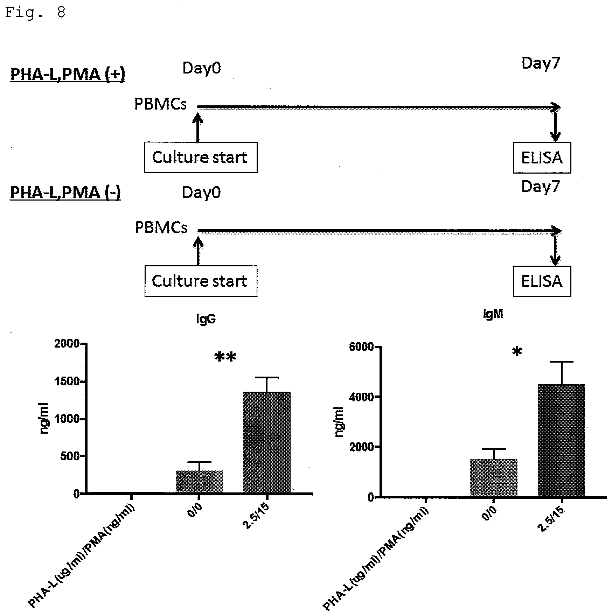

As a result, by adding PHA-L (T cell activation) and PMA (tyrosine kinase activator; activation of inflammatory cells) to the PBMC culture condition, significantly more IgG and IgM could be detected in the culture supernatant (antibodies derived from memory B cells were primarily detected)(Fig. 8).

### [Example 10]

### IgM DSA rather than IgG DSA is detected earlier in culture supernatant also in cases after renal transplantation

Peripheral blood mononuclear cells (5 × 10⁵ cells/well) taken from cases after renal transplantation were cultured on 24-well flat-bottom plates. For cytokines, IL-21 (50 ng/ml), CpG-ODN (2.5 pg/ml), phorbol myristate acetate (PMA)(2.5 pg/ml), phytohemagglutinin/leucoagglutinin (PHA-L)(15 µg/ml) were added as basal culture conditions and the culture was conducted for 7 days. Thereafter, the culture supernatant was collected and, after concentration by 5-fold, analyzed by Flow PRA screening. In positive cases, the specificity of HLA antibodies and the presence and absence of DSA were further evaluated by a single antigen beads method.

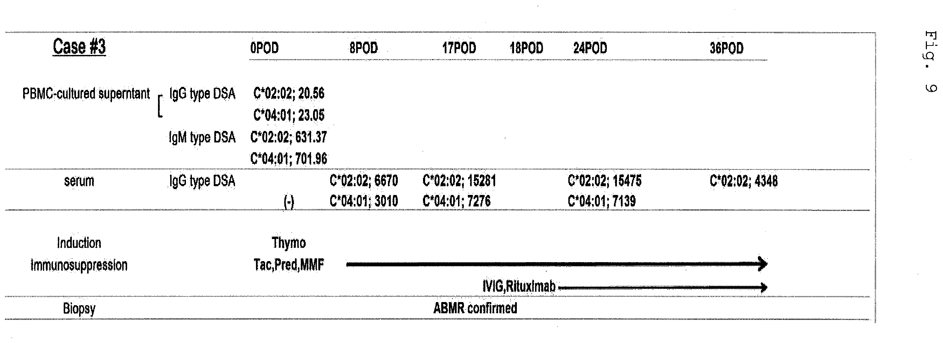

As a result, IgM DSA was detected in the culture supernatant even in cases where IgG DSA was not detected in sera (Figs. 9-11).

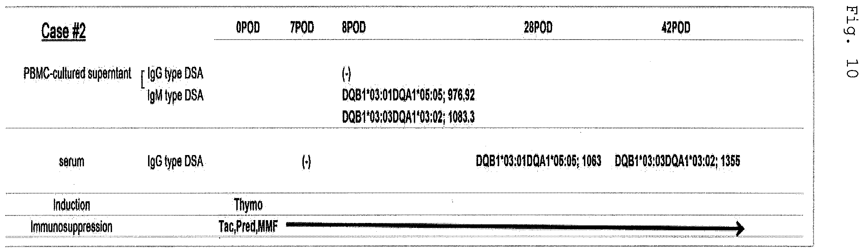

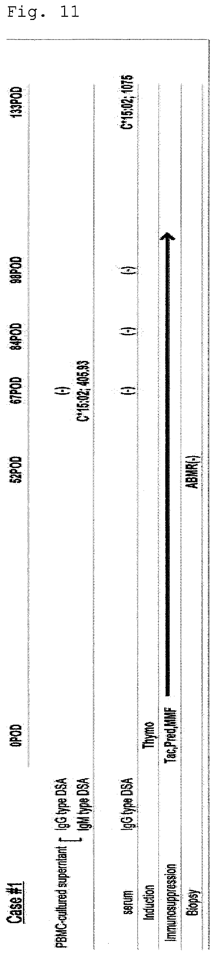

In Fig. 9, IgM DSA was detected in the culture supernatant immediately after transplantation, thereafter IgG DSA was detected in sera on Day 8 after transplantation, and the patient was diagnosed as ABMR on Day 9 after transplantation. In Fig. 10, IgM DSA was detected in PBMC culture supernatant obtained on Day 8 after transplantation and IgG DSA was detected in sera on Day 28 after transplantation. In Fig. 11, IgM DSA was detected in PBMC culture supernatant obtained on Day 67 after transplantation and IgG DSA was detected in sera on Day 133 after transplantation.

### [Example 11]

### DSA-specific IgM memory B cells can be removed by ordinary immunosuppressive therapy but DSA-specific IgG memory B cells needs potent immunosuppressive therapy such as desensitization therapy

In cases where peripheral blood mononuclear cells were taken before transplantation and IgG DSA was detected in culture supernatant (transplant is rejected by primary transplantation) and in cases where peripheral blood mononuclear cells were taken before transplantation and IgM DSA was detected in culture supernatant, peripheral blood mononuclear cells were also taken after transplantation and IgG/IgM DSA transition in the culture supernatant was investigated.

Peripheral blood mononuclear cells (5 × 10⁵ cells/well) taken before a secondary transplantation were cultured on 24-well flat-bottom plates. For cytokines, IL-21 (50 ng/ml), CpG-ODN (2.5 pg/ml), phorbol myristate acetate (PMA)(2.5 µg/ml), phytohemagglutinin/leucoagglutinin (PHA-L) (15 µg/ml) were added as basal culture conditions and the culture was conducted for 7 days. In cases where IgG or IgM antibodies against the primary donor antigen were detected in the culture supernatant, peripheral blood mononuclear cells were taken after transplantation and the culture was conducted similarly. The culture supernatant was analyzed and a detection amount of IgG or IgM antibodies against the primary donor antigen was compared to that obtained before transplantation. At the time of transplantation, medication of standard immunosuppressive agents, thymoglobuline (removal of T cells), predonine, cellcept and tacrolimus was initiated.

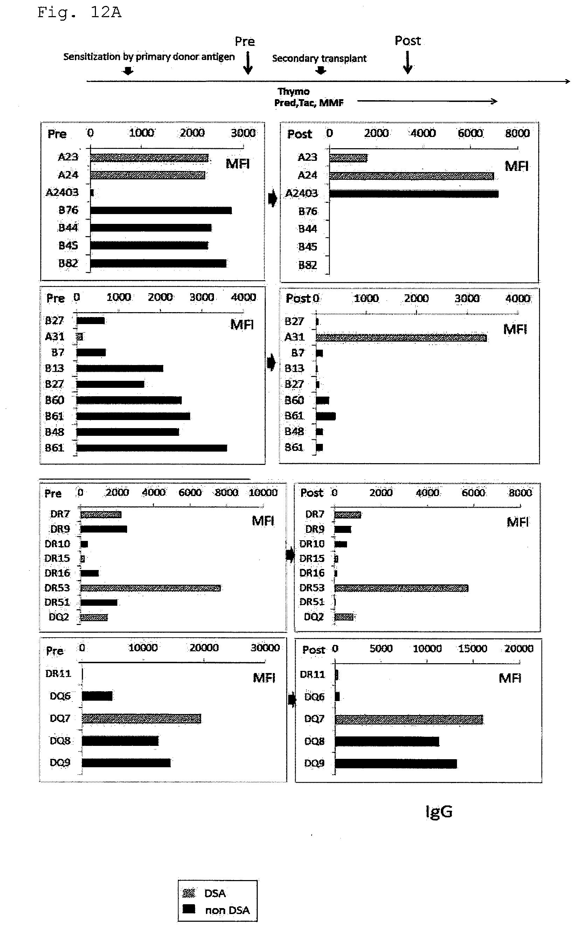

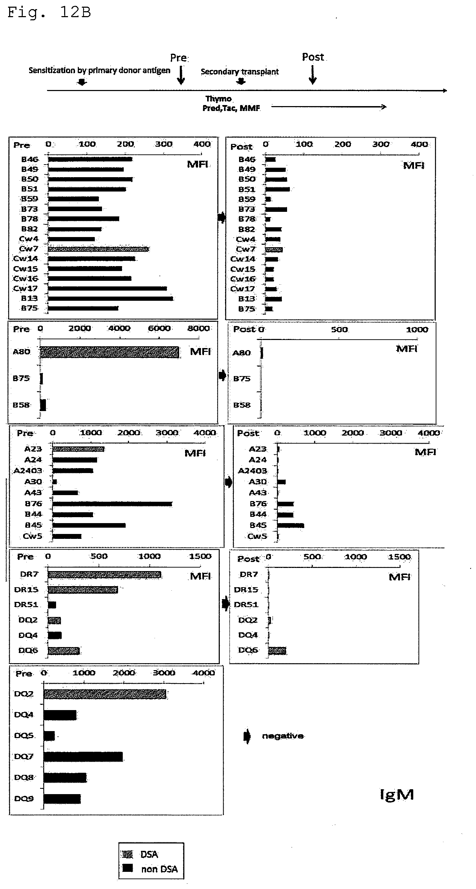

As a result, a detection amount of IgG DSA was not changed before and after transplantation whereas a detection amount of IgM DSA was markedly decreased after transplantation (Fig. 12A, Fig. 12B). As such, it was found that DSA-specific IgM memory B cells could be removed by ordinary immunosuppressive therapy whereas DSA-specific IgG memory B cells needed potent immunosuppressive therapy such as desensitization therapy.

### INDUSTRIAL APPLICABILITY

In accordance with the present invention, the presence or absence of ABMR in patients after organ transplantation can be detected in vitro at an early stage. Biopsy of an organ, the only method for diagnosis of ABMR hitherto performed, was highly invasive and was difficult to perform frequently. In accordance with the present invention, diagnosis of ABMR can be carried out non-invasively with a small amount of peripheral blood.

## Claims

1. A method for detecting in vitro the presence or absence of antibody mediated rejection (hereinafter referred to as "ABMR") in patients after organ transplantation, comprising culturing peripheral blood sample from the patients in the presence of humoral factors from activated T cells and/or inflammatory cytokines, and detecting IgM-type anti-donor HLA antibody (Donor Specific HLA Antibody; hereinafter referred to as "DSA") in the culture supernatant, wherein the detection of IgM-type DSA can be an index of early diagnosis of ABMR.

2. The method of claim 1 wherein culturing in the presence of humoral factors from activated T cells and/or inflammatory cytokines is carried out by addition of healthy adult mononuclear cell culture supernatant.

3. The method of claim 1 or 2 wherein the organ transplantation is renal transplantation.

4. The method of any of claims 1 to 3 wherein culturing peripheral blood sample from the patients is carried out under culture conditions suitable for B cells.

5. A kit for detecting in vitro the presence or absence of antibody mediated rejection (ABMR) in patients after organ transplantation, comprising a means for culturing peripheral blood sample from the patients in the presence of humoral factors from activated T cells and/or inflammatory cytokines, and a means for detecting IgM-type DSA in the culture supernatant, wherein the detection of IgM-type DSA can be an index of the presence of ABMR.

6. The kit of claim 5 wherein the means for culturing peripheral blood sample from the patients in the presence of humoral factors from activated T cells and/or inflammatory cytokines comprises healthy adult mononuclear cell culture supernatant.

7. The kit of claim 5 or 6 wherein the organ transplantation is renal transplantation.

8. The kit of any of claims 5 to 7 wherein the means for culturing peripheral blood sample from the patients is a means for culturing peripheral blood sample from the patients under culture conditions suitable for B cells.
